# EUROPEAN PATENT APPLICATION

(11) **EP 4 811 334 A1**
(43) Date of publication of application: **23.09.2026**
(21) Application number: 26161222.0
(22) Date of filing: 27.02.2026
(51) Int. Cl.: G09F 3/00, G09F 3/02

(54) **SELF-FLAGGING SYRINGE LABEL**

(30) Priority: 27.02.2025 US 202519065941
(71) Applicant: Omnicell, Inc., Fort Worth Texas 76137 (US)
(72) Inventor: DAVID, Ian, Pittsburgh, 15235 (US); SCHWARTZ, Larissa, Santa Barbara, 93109 (US); PUSKAR-PASEWICZ, John, Sharpsburg, 15215 (US)
(74) Representative: Bird & Bird LLP

(57) **Abstract**

A self-flagging label having a transparent adhesive portion configured for adhesion to a barrel of a syringe, an opaque printable portion, and a narrow bridge connecting the transparent adhesive portion to the opaque printable portion, wherein, when the transparent adhesive portion is applied to the barrel of the syringe, the narrow bridge is configured to allow the opaque printable portion to form a hinged connection to the barrel of the syringe such that graduation marks on the barrel of the syringe are unobstructed by the opaque printable portion.

## Description

### TECHNICAL FIELD

Aspects of the present disclosure generally relate to labeling systems for medical syringes, and more particularly, to a self-flagging adhesive label configured to enhance the visibility and accessibility of critical information on syringes.

### BACKGROUND

Reference may be made herein to other United States patents, foreign patents, and/or other technical references. Any reference made herein to other documents is an express incorporation by reference of the document so referenced in its entirety.

In medical practice, the accurate administration of medications via syringes relies heavily on the visibility and accessibility of essential information, including dosage, medication identity, expiration dates, etc. Conventional syringe labeling systems have been developed to meet these needs. However, these systems present significant limitations that can impede efficient and safe medication administration.

One prominent issue with current syringe labeling systems is their potential to obstruct the graduation marks on the syringe barrel. For example, conventional multi-layer label designs feature a clear, adhesive layer that attaches directly to the syringe surface and an opaque layer, containing the actual label content, hinging off the clear layer. Upon initial application of the label, this opaque layer obscures the graduation marks on the barrel of the syringe. To access the graduation marks, an end-user must "flag out" or pull back the opaque layer, revealing the markings beneath. However, this process often requires the user to defeat a small amount of light-duty adhesive, which can be cumbersome and time-consuming, particularly in fast-paced clinical environments, and often leaves adhesive residue on the syringe further obscuring the graduation marks. Moreover, if the hinged connection between the layers ends up misaligned on the barrel of the syringe, the graduation marks may remain obscured, leading to potential dosage errors and compromised patient safety.

Other, traditional syringe labels may include, for example, an entirely opaque flagging label having a thin adhesive portion adhering to the syringe barrel so as to avoid obstruction of the syringe graduation marks. The reduced surface area of the adhesive portion limits the effectiveness of adhesion, and may increase the likelihood of label detachment or misalignment during handling and use, especially with smaller diameter syringe barrels.

Furthermore, conventional syringe labeling systems often suffer from limitations in information presentation. The restricted surface area of the label may necessitate small font sizes or abbreviated information, making it difficult for healthcare professionals to quickly locate and interpret the relevant details, especially in high-stress clinical scenarios.

Where several different materials are used in a single label, there may also be an increase in manufacturing costs. For example, with a rectangular label requiring a clear, adhesive portion followed by a printable, opaque portion, it is economically and logistically problematic to utilize a label roll with alternating adhesive and non-adhesive portions along the rolling direction. Several conventional syringe label manufacturers thus arrange labels "across" the label roll to alleviate this issue, wherein the label roll may have a continuous adhesive portion adjacent to a continuous non-adhesive portion such that a conventional, rectangular label is printed having both adhesive and non-adhesive portions along the rolling direction. However, this approach poses challenges in terms of unrolling, printing, and applying the label to a cylindrical syringe barrel parallel to the label roll and often requires manual application of syringe labels after printing.

These challenges underscore the critical need for an innovative syringe labeling solution that overcomes the limitations of current systems, enhances the visibility and accessibility of critical information, and ensures accurate and efficient medication administration in healthcare settings.

### SUMMARY

Techniques described herein are directed to a self-flagging syringe label for enhanced visibility and accessibility of critical information on syringes, and syringe labeling systems for applying the self-flagging syringe label to syringes. The self-flagging syringe label disclosed herein is a label that has separate adhesive and non-adhesive portions connected by a bridge that when applied to a syringe allows the user of the syringe to easily pull back the non-adhesive portion of the label from the syringe when needed. The self-flagging syringe label disclosed herein also allows for ease of automated application by providing the adhesive portion with a continuous leading edge that can be applied to, for example, a syringe barrel. An example embodiment of the present disclosure may be a self-flagging label comprising: a transparent adhesive portion configured for adhesion to a barrel of a syringe; an opaque printable portion; and a narrow bridge connecting the transparent adhesive portion to the opaque printable portion; wherein, when the transparent adhesive portion is applied to the barrel of the syringe, the narrow bridge is configured to allow the opaque printable portion to form a hinged connection to the barrel of the syringe such that graduation marks on the barrel of the syringe are unobstructed by the opaque printable portion.

In one embodiment, the transparent adhesive portion, the opaque printable portion, and the narrow bridge are in a C-shaped configuration.

In one embodiment, the transparent adhesive portion, the opaque printable portion, and the narrow bridge are in a backwards Z-shaped configuration.

In one embodiment, the opaque printable portion is laterally disposed from the transparent adhesive portion.

In one embodiment, information is printed on the opaque printable portion in a vertical orientation.

In one embodiment, information is printed on the opaque printable portion in a horizontal orientation.

In one embodiment, the transparent adhesive portion is composed of a semi-transparent material.

In one embodiment, the transparent adhesive portion is the same width as the opaque printable portion.

In one embodiment, the transparent adhesive portion is a different width than opaque printable portion.

In one embodiment, a first portion of the narrow bridge comprises transparent material, and a second portion of the narrow bridge comprises opaque material.

Another example embodiment of the present disclosure may be an automated syringe label application system comprising: an application device; and a self-flagging label roll comprising one or more self-flagging labels, each of the one or more self-flagging labels, comprising: a transparent adhesive portion configured for adhesion to a barrel of a syringe; an opaque printable portion; and a narrow bridge connecting the transparent adhesive portion to the opaque printable portion; wherein the application device is configured to apply a self-flagging label of the one or more self-flagging labels to the barrel of the syringe, wherein the narrow bridge is configured to allow the opaque printable portion to form a hinged connection to the barrel of the syringe such that graduation marks on the barrel of the syringe are unobstructed by the opaque printable portion.

In one embodiment, the transparent adhesive portion, the opaque printable portion, and the narrow bridge are configured in a C-shape.

In one embodiment, the transparent adhesive portion, the opaque printable portion, and the narrow bridge are configured in a backwards Z-shape.

In one embodiment, the opaque printable portion is laterally disposed from the transparent adhesive portion.

In one embodiment, information is printed on the opaque printable portion in a vertical orientation.

In one embodiment, information is printed on the opaque printable portion in a horizontal orientation.

In one embodiment, the transparent adhesive portion is composed of a semi-transparent material.

In one embodiment, the transparent adhesive portion is the same width as the opaque printable portion.

In one embodiment, the transparent adhesive portion is a different width than opaque printable portion.

In one embodiment, a first portion of the narrow bridge comprises transparent material, and a second portion of the narrow bridge comprises opaque material.

The above summary has outlined, rather broadly, some features and technical advantages of the present disclosure so that the detailed description that follows may be better understood. Additional features and advantages of this disclosure will be described below. It should be appreciated by those skilled in the art that this disclosure may be readily utilized as a basis for modifying or designing other systems and structures for carrying out the same or similar purposes of the present disclosure. It should also be realized by those skilled in the art that such equivalent systems and structures do not depart from the teachings of the disclosure as set forth in the appended claims. The novel features, which are believed to be characteristic of this disclosure, both as to its organization and method of operation, together with further features and advantages, will be better understood from the following description when considered in connection with the accompanying figures. It is to be expressly understood, however, that each of the figures is provided for the purpose of illustration and description only and is not intended as a definition of the limits of the present disclosure.

### BRIEF DESCRIPTION OF THE DRAWINGS

The features, nature, and advantages of the present disclosure will become more apparent from the detailed description set forth below when taken in conjunction with the drawings, in which:
FIG. 1 depicts an illustrative example of a first embodiment of a self-flagging syringe label;
FIG. 2 depicts an illustrative example of a second embodiment of a self-flagging syringe label;
FIG. 3 depicts an illustrative example of a self-flagging syringe label securely applied to a syringe barrel;
FIG. 4 depicts an illustrative example of a self-flagging syringe label having vertical text; and
FIG. 5 depicts an illustrative example of a self-flagging syringe label in a rolled configuration, ready for printing and application to a syringe.

### DETAILED DESCRIPTION

The detailed description set forth below, in connection with the appended drawings, is intended as a description of various configurations and is not intended to represent the only configurations in which the concepts described herein may be practiced. The detailed description includes specific details for the purpose of providing a thorough understanding of the various concepts disclosed herein. It will be apparent, however, to those skilled in the art that these concepts may be practiced without these specific details. In some instances, well-known features may be omitted or simplified in order to not obscure the relevant features of the embodiment being described.

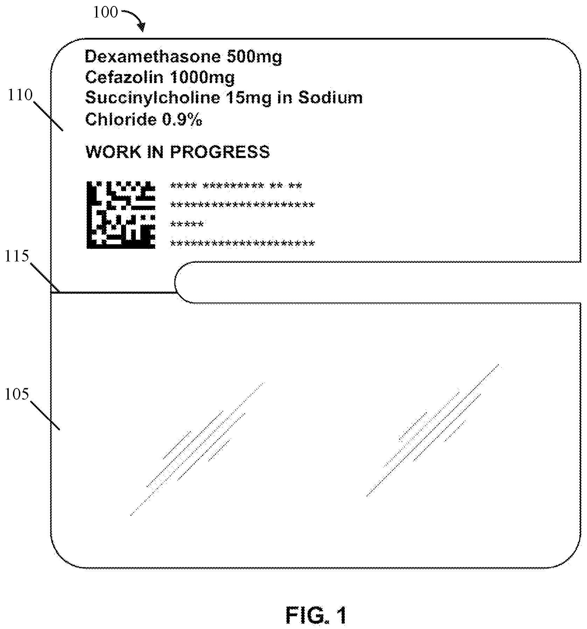

FIG. 1 illustrates a first exemplary embodiment of a self-flagging syringe label 100. Label 100 includes a "C-shaped" design having transparent adhesive portion 105 and opaque printable portion 110 connected by bridge 115, forming the distinctive C-shape. The printable area of opaque printable portion 110 is laterally disposed from the adhesive area of transparent adhesive portion 105, facilitating printing and application processes.

Transparent adhesive portion 105 is configured for application to, for example, a barrel of a syringe, and may be composed of any clear, adhesive material known in the art. In a preferred embodiment, transparent adhesive portion 105 may be a double layer polyethylene terephthalate (PET) film having a pressure sensitive acrylic adhesive and a clear PET liner. Additionally, transparent adhesive film may be a semi-transparent material.

Opaque printable portion 110 may include printable text for display to a user without obstruction of the syringe barrel. In the present embodiment, the printable text is displayed horizontally. However, as will be appreciated by one of skill in the art, the printable text may be displayed in other orientations, such as vertically, diagonally, etc. Opaque printable portion 110 may be composed of any suitable material known in the art. In a preferred embodiment, opaque printable portion 110 may be a phenol-free matte white polypropylene film coated for direct thermal printing and having a pressure sensitive acrylic adhesive.

Bridge 115 is an area between transparent adhesive portion 105 and opaque printable portion 110 and may be composed of transparent material, opaque material, or a combination of transparent and opaque material. Bridge 115 acts as a hinge between transparent adhesive portion 105 and opaque printable portion 110 ensuring that graduation marks of the syringe barrel are visible regardless of syringe barrel diameter and orientation of label 100. For example, with this configuration printable portion 110 is able to easily pulled away from the barrel of the syringe by a user to allow the user to view the graduation marks on the syringe barrel when needed. While a narrow bridge is depicted, it should be appreciated by one of skill in the art that bridge 115 may be of different widths depending on desired characteristics of label 100. For example, a thinner width may require less material, may provide more flexibility to label 100 when applied and flagged, but may be less durable than bridge 115 having a thicker width. Additionally, transparent adhesive portion 105 may be the same width as opaque printable portion 110.

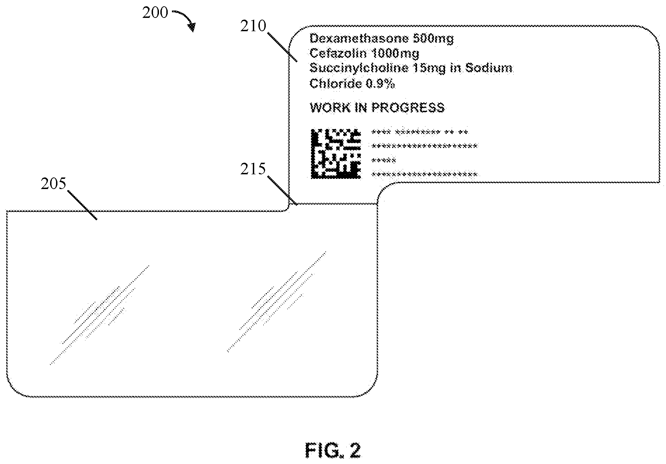

FIG. 2 illustrates a second exemplary embodiment of a self-flagging syringe label 200. Label 200 includes a reverse or backwards "Z-shaped" design having transparent adhesive portion 205 and opaque printable portion 210 connected by bridge 215, forming the distinctive reverse Z-shape. It should be appreciated that label 200 may also be in a "Z-shaped" design. The printable area of opaque printable portion 210 is laterally disposed from the adhesive area of transparent adhesive portion 205, facilitating printing and application processes.

Transparent adhesive portion 205 is configured for application to, for example, a barrel of a syringe, and may be composed of any clear, adhesive material known in the art. In a preferred embodiment, transparent adhesive portion 205 may be a double layer polyethylene terephthalate (PET) film having a pressure sensitive acrylic adhesive and a clear PET liner. Additionally, transparent adhesive portion 205 may be a semi-transparent material.

Opaque printable portion 210 may include printable text for display to a user without obstruction of the syringe barrel. Opaque printable portion 210 may be composed of any suitable material known in the art. In a preferred embodiment, opaque printable portion 210 may be a phenol-free matte white polypropylene film coated for direct thermal printing and having a pressure sensitive acrylic adhesive.

Bridge 215 is an area between transparent adhesive portion 205 and opaque printable portion 210 and may be composed of transparent material, opaque material, or a combination of transparent and opaque material. Bridge 215 acts as a hinge between transparent adhesive portion 205 and opaque printable portion 210 ensuring that graduation marks of the syringe barrel are visible regardless of syringe barrel diameter and orientation of label 200. For example, with this configuration printable portion 110 is able to easily pulled away from the barrel of the syringe by a user to allow the user to view the graduation marks on the syringe barrel when needed. While a narrow bridge is depicted, it should be appreciated by one of skill in the art that bridge 215 may be of different widths depending on desired characteristics of label 200. For example, a thinner width may require less material, may provide more flexibility to label 200 when applied and flagged, but may be less durable than bridge 215 having a thicker width. Additionally, transparent adhesive portion 205 may be the same width as opaque printable portion 210.

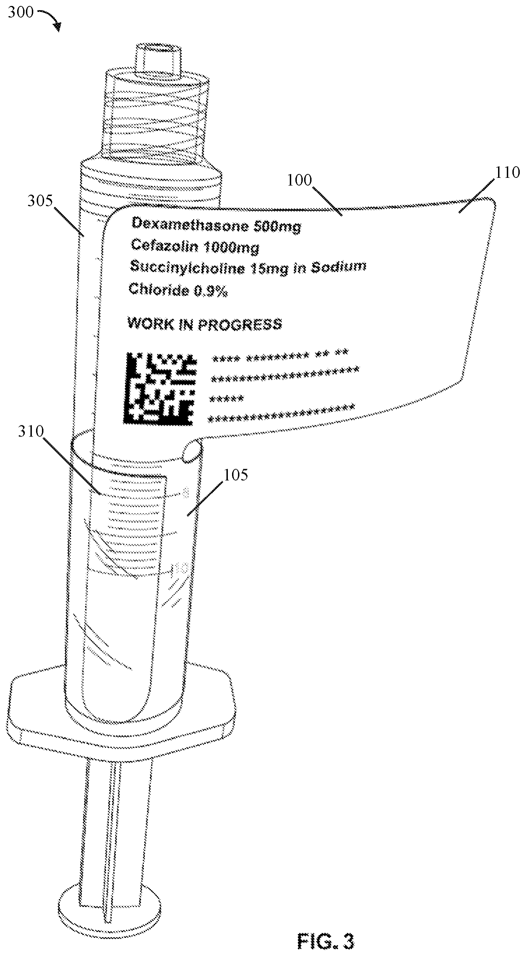

FIG. 3 illustrates the self-flagging syringe label 100 of FIG. 1 securely applied to a syringe barrel 305. Syringe barrel 305 of syringe 300 may include graduation marks 310 to indicate an amount of medication stored within syringe 300. As will be appreciated by one of skill in the art, syringe barrel 305 may be of various different diameters based on, for example, amount of medication to be injected, desired flow rate, etc. Regardless of syringe barrel diameter, transparent adhesive portion 105 is configured for adherence to syringe barrel 305 without obstruction of graduation marks 310. While not necessary, transparent adhesive portion 105 may be trimmed prior to application for syringes having a narrow diameter such that wraparound of transparent adhesive portion 105 is minimized. Label 100 may also be printed with a shorter length transparent adhesive portion 105 depending on the syringe to which label 100 will be applied. Narrow bridge 115 between transparent adhesive portion 105 and the opaque printable portion 110 allows for self-flagging of opaque printable portion 110 such that visibility of graduation marks 310 is unobstructed because the opaque printable portion 110 may be easily pulled away from the syringe.

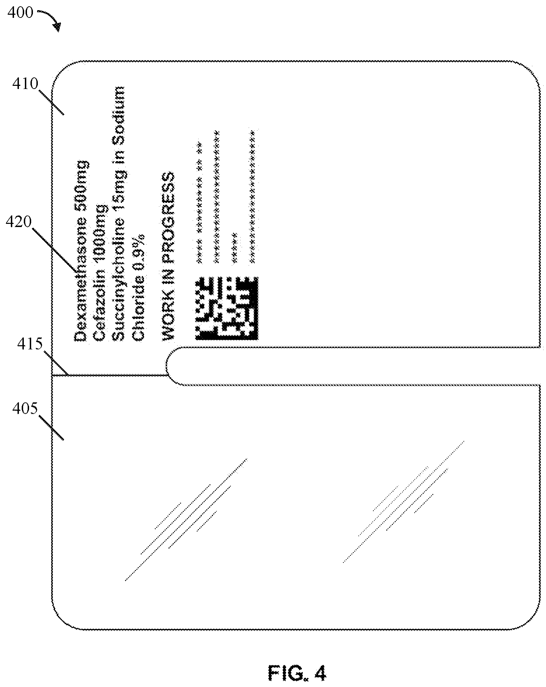

FIG. 4 illustrates an exemplary label 400 having vertical text. Label 400 is similar to label 100 in that it comprises a transparent adhesive portion 405 and an opaque printable portion 410 connected by a bridge 415, forming a C-shape. Label 400 is adapted to display information 420, and may accommodate diverse content requirements, such as, without limitation, medication name, dosage, expiration date, etc., ensuring clarity and readability in medical settings. Depending on the content of information 420 and dimensions of label 400, information 420 may be displayed in a vertical orientation, a horizontal orientation, a diagonal orientation, etc.

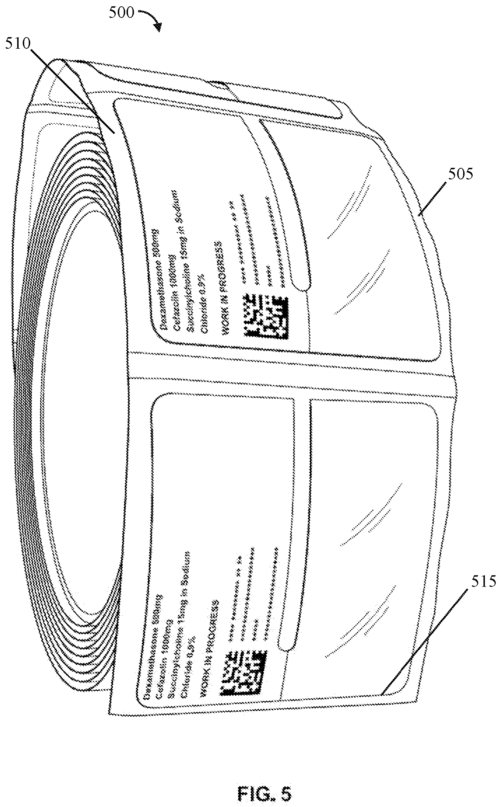

FIG. 5 illustrates the self-flagging syringe label 100 from Fig. 1 in a rolled configuration, ready for printing and application. Shown in Fig. 5 is roll 500, which may include adhesive portion 505 and non-adhesive portion 510. Adhesive portion 505 includes a continuous leading edge 515, allowing for smooth feeding of roll 500 through a printing and/or application apparatus, optimizing efficiency in large-scale production.

The label described in the embodiments above may have information and/or text printed on it during manufacturing of the roll, or alternatively a roll of blank labels may be manufactured and text may be applied to the label at the point of application of the label on the syringe using any method known to a person of skill in the art for applying text to the label. A manufacturing system may be used for production of the label. The system includes components for printing, cutting, and rolling labels onto rolls, streamlining production processes and maintaining product quality.

A system may also be used for the automatic application of the label to, for example, the body or barrel of a syringe. The system for automatic application may feature mechanisms for precise label placement on syringe barrels, minimizing human error and optimizing workflow efficiency in medical facilities. The system may also include one or more rollers for rotating the syringe and a label application system for applying the label to the syringe barrel. Because of the continuous leading edge of the adhesive portion of each label, labels may be continuously applied to syringes without adjustment of the label application system. That is, the label may be positioned on the label roll such that the label includes sufficient length perpendicular to the syringe barrel to allow for the transparent adhesive portion to be grabbed and rolled around the syringe barrel. Thus, the label roll may be utilized in an industrial process without the need for manual flagging of opaque printable portion.

The specification and drawings are, accordingly, to be regarded in an illustrative rather than a restrictive sense. It will, however, be evident that various modifications and changes may be made thereunto without departing from the broader spirit and scope of the disclosure as set forth in the claims.

Other variations are within the spirit of the present disclosure. Thus, while the disclosed techniques are susceptible to various modifications and alternative constructions, certain illustrated embodiments thereof are shown in the drawings and have been described above in detail. It should be understood, however, that there is no intention to limit the disclosure to the specific form or forms disclosed, but on the contrary, the intention is to cover all modifications, alternative constructions, and equivalents falling within the spirit and scope of the disclosure, as defined in the appended claims.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including," and "containing" are to
be construed as open-ended terms (i.e., meaning "including, but not limited to,") unless otherwise noted. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate embodiments of the disclosure and does not pose a limitation on the scope of the disclosure unless otherwise claimed. No language in the specification should be construed as indicating any non-claimed element as essential to the practice of the disclosure

Disjunctive language such as the phrase "at least one of X, Y, or Z," unless specifically stated otherwise, is intended to be understood within the context as used in general to present that an item, term, etc., may be either X, Y, or Z, or any combination thereof (e.g., X, Y, and/or Z). Thus, such disjunctive language is not generally intended to, and should not, imply that certain embodiments require at least one of X, at least one of Y, or at least one of Z to each be present

Although the present disclosure and its advantages have been described in detail, it should be understood that various changes, substitutions and alterations can be made herein without departing from the technology of the disclosure as defined by the appended claims. For example, relational terms, such as "above" and "below" and/or "inside" and "outside" are used with respect to a specific device. Of course, if the device is inverted, above becomes below, and vice versa. Additionally, if oriented sideways, above and below may refer to sides of a device. Further, reference to "first" or "second" instances of a feature, element, or device does not indicate that one device comes before or after the other listed device. Reference to first and/or second devices merely serves to distinguish one device that may be similar or similarly referenced with respect to another device.

Moreover, the scope of the present application is not intended to be limited to the particular configurations of the process, machine, manufacture, composition of matter, means, methods and steps described in the specification. As one of ordinary skill in the art will readily appreciate from the disclosure, processes, machines, manufacture, compositions of matter, means, methods, or steps, presently existing or later to be developed that perform substantially the same function or achieve substantially the same result as the corresponding configurations described herein may be utilized according to the present disclosure. Accordingly, the appended claims are intended to include within their scope such processes, machines, manufacture, compositions of matter, means, methods, or steps.

The description of the disclosure is provided to enable any person skilled in the art to make or use the disclosure. Various modifications to the disclosure will be readily apparent to those reasonably skilled in the art, and the generic principles defined herein may be applied to other variations without departing from the spirit or scope of the disclosure. Thus, the present disclosure is not intended to be limited to the examples and designs described herein but is to be accorded the widest scope consistent with the principles and novel features disclosed herein. Accordingly, the disclosure is not to be limited by the examples presented herein, but is envisioned as encompassing the scope described in the appended claims and the full range of equivalents of the appended claims.

## Claims

1. A self-flagging label, comprising:
a transparent adhesive portion configured for adhesion to a barrel of a syringe;
an opaque printable portion; and
a narrow bridge connecting the transparent adhesive portion to the opaque printable portion;
wherein, when the transparent adhesive portion is applied to the barrel of the syringe, the narrow bridge is configured to allow the opaque printable portion to form a hinged connection to the barrel of the syringe such that graduation marks on the barrel of the syringe are unobstructed by the opaque printable portion.

2. The self-flagging label of claim 1, wherein the transparent adhesive portion, the opaque printable portion, and the narrow bridge are in a C-shaped configuration.

3. The self-flagging label of claim 1, wherein the transparent adhesive portion, the opaque printable portion, and the narrow bridge are in a backwards Z-shaped configuration.

4. The self-flagging label of claim 1, wherein the opaque printable portion is laterally disposed from the transparent adhesive portion.

5. The self-flagging label of claim 1, wherein information is printed on the opaque printable portion in a vertical orientation.

6. The self-flagging label of claim 1, wherein information is printed on the opaque printable portion in a horizontal orientation.

7. The self-flagging label of claim 1, wherein the transparent adhesive portion is composed of a semi-transparent material.

8. The self-flagging label of claim 1, wherein the transparent adhesive portion is the same width as the opaque printable portion.

9. The self-flagging label of claim 1, wherein the transparent adhesive portion is a different width than opaque printable portion.

10. The self-flagging label of claim 1, wherein a first portion of the narrow bridge comprises transparent material, and a second portion of the narrow bridge comprises opaque material.

11. An automated syringe label application system, comprising;
an application device; and
a self-flagging label roll comprising one or more self-flagging labels, each of the one or more self-flagging labels, comprising:
a transparent adhesive portion configured for adhesion to a barrel of a syringe;
an opaque printable portion; and
a narrow bridge connecting the transparent adhesive portion to the opaque printable portion;
wherein the application device is configured to apply a self-flagging label of the one or more self-flagging labels to the barrel of the syringe,
wherein the narrow bridge is configured to allow the opaque printable portion to form a hinged connection to the barrel of the syringe such that graduation marks on the barrel of the syringe are unobstructed by the opaque printable portion.

12. The automated syringe label application system of claim 11, wherein the transparent adhesive portion, the opaque printable portion, and the narrow bridge are configured in a C-shape.

13. The automated syringe label application system of claim 11, wherein the transparent adhesive portion, the opaque printable portion, and the narrow bridge are configured in a backwards Z-shape.

14. The automated syringe label application system of claim 11, wherein the opaque printable portion is laterally disposed from the transparent adhesive portion.

15. The automated syringe label application system of claim 11, wherein information is printed on the opaque printable portion in a vertical orientation.

16. The automated syringe label application system of claim 11, wherein information is printed on the opaque printable portion in a horizontal orientation.

17. The automated syringe label application system of claim **11,** wherein the transparent adhesive portion is composed of a semi-transparent material.

18. The automated syringe label application system of claim **11,** wherein the transparent adhesive portion is the same width as the opaque printable portion.

19. The automated syringe label application system of claim **11,** wherein the transparent adhesive portion is a different width than opaque printable portion.

20. The automated syringe label application system of claim **11,** wherein a first portion of the narrow bridge comprises transparent material, and a second portion of the narrow bridge comprises opaque material.
